## Europäisches Patentamt
## European Patent Office
## Office européen des brevets

(11) Veröffentlichungsnummer: **0 157 172**
**B1**

(12) # EUROPÄISCHE PATENTSCHRIFT

(45) Veröffentlichungstag der Patentschrift:
14.10.87

(21) Anmeldenummer: **85102147.7**

(22) Anmeldetag: **27.02.85**

(51) Int. Cl.⁴: **A 61 B 17/16, B 25 D 3/00**

(54) **Chirurgischer Meissel.**

(30) Priorität: **05.03.84 DE 8406730 U**

(43) Veröffentlichungstag der Anmeldung:
**09.10.85 Patentblatt 85/41**

(45) Bekanntmachung des Hinweises auf die Patenterteilung:
**14.10.87 Patentblatt 87/42**

(84) Benannte Vertragsstaaten:
**CH DE FR GB IT LI NL SE**

(56) Entgegenhaltungen:
**WO-A-82/02328**
**CH-A-405 193**
**DE-C-129 099**
**FR-A-964 389**
**FR-A-1 494 869**

(73) Patentinhaber: **Waldemar Link GmbH & Co,**
**Barkhausenweg 10, D-2000 Hamburg 63 (DE)**

(72) Erfinder: **Keller, Arnold, An der Naherfurth 5,**
**D-2061 Kayhude (DE)**

(74) Vertreter: **Glawe, Delfs, Moll & Partner**
**Patentanwälte, Postfach 26 01 62 Liebherrstrasse**
**20, D-8000 München 26 (DE)**

LIBER, STOCKHOLM 1987

## Beschreibung

Meißel sind in der Knochenchirurgie und Orthopädie häufig gebrauchte Instrumente für verschiedene Trennzwecke, z. B. für Osteotomie oder für das Abmeißeln von Lamellen. Sie müssen eine beträchtliche Stabilität, insbesondere gegenüber Knickbeanspruchung, aufweisen, die für eine gegebene Meißelbreite durch die Materialdicke bestimmt ist. Die Dicke muß daher um so größer sein, je größer die Meißellänge und die zu erwartende Beanspruchung sind. Immer wenn lange Strecken zu durchtrennen sind, beispielsweise bei Osteotomien, Meißeln im oberen Schenkel-Kopfbereich, Abmeißeln der Tibia-Gelenkfläche, entstehen für den Operateur bei Benutzung herkömmlicher Meißel nicht unerhebliche Schwierigkeiten, infolge des großen Meißelquerschnitts, nämlich insbesondere die Gefahr einer vorzeitigen und ggf. unkontrollierten Frakturierung des Knochens, zu starke Traumatisierung des Knochens sowie die Gefahr des Einklemmens des Meißels, der sich dann in der Regel nur schwer entfernen läßt.

Der Erfindung liegt die Aufgabe zugrunde, diese Nachteile zu vermindern.

Die erfindungsgemäße Lösung besteht darin, daß am vorderen Ende eines biegestarren Meißelschafts eine langgestreckte, dünne, an ihrem vorderen Ende die Meißelschneide tragende Meißelklinge richtungsgleich befestigt ist und daß der Meißelschaft und die Meißelklinge in Gleitführungen einer biegestarren Meißelführung in ihrer Längsrichtung verschiebbar geführt sind.

Da die Meißelklinge dank der Führung sehr dünn sein kann, z. B. 0,5 oder 1,0 oder 1,5 oder 2 mm, findet nur eine geringe Aufweitung des Schnittspalts durch die Meißeldicke statt. Die Gefahr vorzeitiger Frakturierung und des Einklemmens wird dadurch herabgesetzt. Die Traumatisierung des Knochens wird vermindert. Dennoch hat der Meißel die erforderliche Stabilität, weil zum einen der Meißelschaft in der Meißelführung sicher geführt ist und daher beim Aufschlagen nicht ausweichen kann und weil zum anderen die Meißelklinge in der Meißelführung vor Knickung geschützt ist und auch in dem durchzutrennenden Gegenstand innerhalb des Schnittspalts so geführt ist, daß sie nicht knicken kann. Dabei ist die Meißelführung zweckmäßigerweise in der Art einer Scheide gestaltet, aus deren vorderem, schmalen Ende die Klinge austritt. Diese Formgebung hat den Vorteil, daß die Meißelführung bis an den durchzutrennenden Gegenstand herangeführt werden kann, so daß die Meißelklinge an keiner Stelle ungeführt der Knickgefahr ausgesetzt ist. Einen geraden Schnitt kann die erfindungsgemäße, dünne Klinge zwar nicht aufgrund ihrer Biegefestigkeit garantieren, wie es bei herkömmlichen Meißeln der Fall ist; jedoch ergibt sich ein gerader Schnitt, wenn die Schneide exakt zur Mitte hin geschliffen ist und damit auf der obere und der unter Flachseite identisch gestaltet ist. Die erfindungsgemäße Klinge ermöglicht gewünschtenfalls auch einen Schnitt gemäß gekrümmter Bahn, nämlich durch unsymmetrischen Schliff.

Der erfindungsgemäße Meißel kann auch mit besonderem Vorteil für sehr tiefe Arbeiten verwendet werden, ohne daß die bei der Verwendung von herkömmlichen Meißeln bekannten Schwierigkeiten in Kauf genommen werden müssen. Die Breite der Meißelklinge kann beliebig bemessen sein.

Die Meißelführung kann dieselben Führungsorgane für die Meißelklinge und den Meißelschaft aufweisen, nämlich einander gegenüberliegende Nuten, in denen die Ränder der Klinge sowie entsprechende Vorsprünge des Meißelschafts verschiebbar sind. Zweckmäßiger sind jedoch gesonderte Gleitführungen für den Meißelschaft und die Meißelklinge.

Vorteilhaft ist es, wenn der Meißel in der Führung arretierbar ist. Dadurch läßt sich die gewünschte Schnittiefe einstellen, indem man die Länge der aus der Führung hervorstehenden Meißelklinge entsprechend einstellt. Dies ist z. B. dann nützlich, wenn ein totales Durchtrennen nicht gewollt ist. Ferner kann dies zweckmäßig sein, wenn der Meißel an der Meißelführung aus dem Knochen herausgezogen werden soll. Dadurch wird die Klinge beim Herausziehen aus dem Knochen weniger offengelegt und die Gefahr einer ungewollten Verbiegung der Klinge verringert.

Für das Einbringen von Löchern in Wände zum Zwecke von elektrischen Installationen ist ein Meißel bekannt, der von einer Hülse umgeben ist, deren Stirnplatte gegen Federkraft entlang dem Meißel verschiebbar ist (DE-A 129 099). Die um den Meißel herum an der zu bearbeitenden Wand unter der Federkraft anliegende Stirnplatte soll das Ausbröckeln von verdrängtem Verputz verhindern.

Zum Halten eines pneumatisch mit hoher Frequenz vorgetriebenen Meißels ist es bekannt (WO-A 82/02 328), am Meißel eine von diesem vibrationsisolierte Hülse anzubringen. Der Meißel ist als solcher biegestarr und wird nicht gegenüber den Knickkräften von der Hülse geführt.

Bei einem anderen bekannten Meißel (CH-A 405 193) ist der Meißel von einer Hülle aus zähem, schlagfestem Kunststoff umgeben, welche den Stahlkern seitlich abstützt und isoliert und die durch den Schlag erzeugten Vibrationen dämpft. Der Querschnitt des Stahlkerns kann sich von dem Schlagkopf und dem Arbeitsende aus nach der Mitte vergrößern nach Art eines schlanken Doppelkonus, wodurch die Sicherheit gegen Knickungsbruch erhöht werden soll. Vergleicht man die Querschnittsabmessungen des Stahlkerns mit denjenigen der Kunststoffhülle und zieht man die unterschiedlichen Elastizitätsmoduln in Betracht, so kann die Vergrößerung des Knickwiderstands durch die Kunststoffhülle nicht nennenswert sein. Die Hülle ist auf dem Meißel nicht verschiebbar.

Zum Stützen eines dünnen, stahldrahtartigen Durchschlags ist es bekannt (FR-A 964 389), diesen verschiebbar in einer Hülse zu führen, die aus mehreren beweglichen Teilen besteht. Im vordersten Abschnitt hat die Hülse einen Durchmesser, der nicht wesentlich größer als der Draht ist. Dahinter bildet sie einen größeren Durchmesser zur Führung von den Draht in einer entsprechenden Bohrung aufnehmenden, verschiebbaren Führungsteilen, die gegeneinander durch Druckfedern abgestützt sind, um stets auch beim Vorbewegen des Drahts einen gleichmäßigen Abstand zur optimaler Abstützung des Drahtes voneinander zu haben.

Demgegenüber hat man bei chirurgischen Meißeln (FR-A 1 494 869) selbst bei der Notwendigkeit schlanken Schnitts stets in sich biegesteife Meißel verwendet.

Die Erfindung wird im folgenden näher unter Bezugnahme auf die Zeichnung erläutert, die vorteilhafte Ausführungsbeispiele veranschaulicht. Darin zeigen:

Fig. 1 eine Seitenansicht des Instruments,

Fig. 2 eine Seitenansicht in Blickrichtung II der Fig. 1,

Fig. 3 und 4 Seitenansicht des Meißels in Darstellungen, die den Fig. 1 und 2 entsprechen,

Fig. 5 eine der Fig. 2 entsprechenden Seitenansicht der Führung,

Fig. 6 eine Schnittansicht gemäß Linie VI-VI der Fig. 5,

Fig. 7 eine der Fig. 1 entsprechende Seitenansicht eines Instruments mit Arretierungseinrichtung,

Fig. 8 und 9 die Arretierungseinrichtung in zwei senkrecht zueinander stehenden Ansichten in vergrößertem Maßstab,

Fig. 10 und 11 Ansichten einer anderen Ausführungsform des Meißels in den Fig. 3 und 4 entsprechenden Darstellungen,

Fig. 12 eine Seitenansicht der zu dieser Ausführung gehörigen Meißelführung und

Fig. 13 eine Endansicht der Meißelführung gemäß Fig. 12.

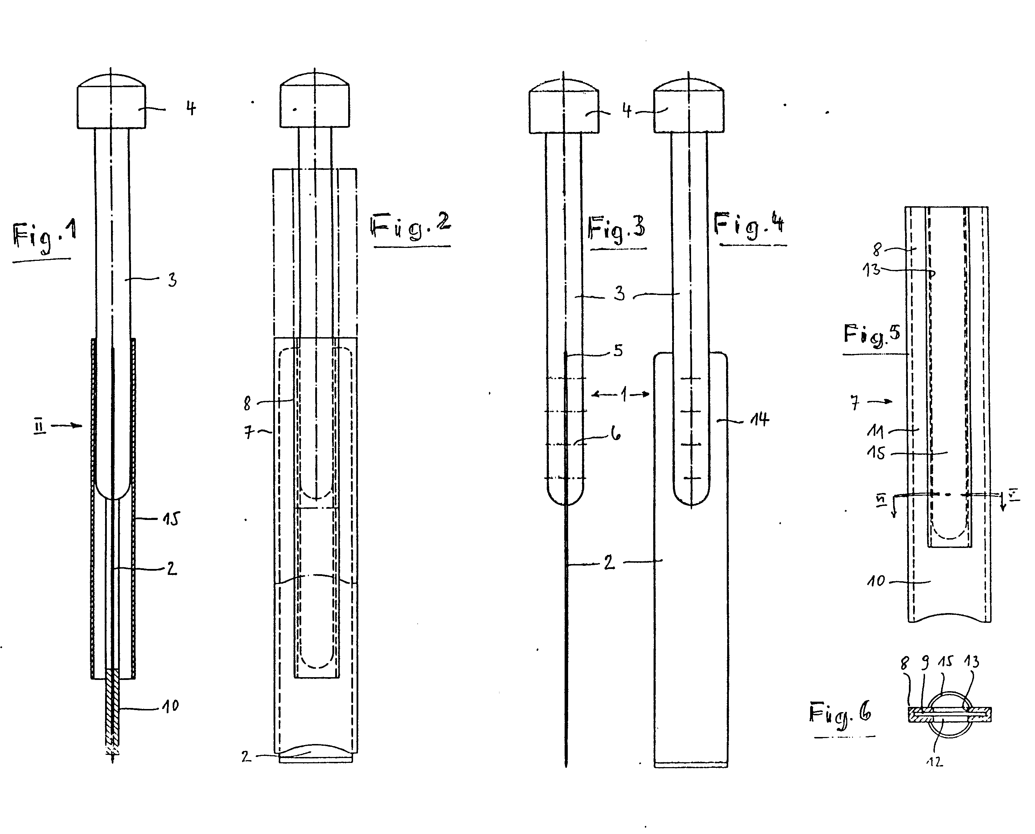

In der Ausführungsform gemäß Fig. 1 bis 6 besteht der Meißel 1 aus der Meißelklinge 2, die von einem Metallblatt vorzugsweise konstanter Dicke und Breite gebildet ist, und dem hinteren Meißelschaft 3, der von einer zylindrischen Stange mit Schlagkopf 4 gebildet ist und an seinem vorderen Ende einen zur Dicke der Meißelklinge 2 passenden Mittellängsschlitz 5 enthält, in welchem die Klinge 2 mittig eingesetzt und durch Schrauben 6 befestigt ist, die durch strichpunktierte Linien angedeutet sind. Die Dicke der Meißelklinge liegt bei Ausführung aus Stahl oder stahlähnlichem Werkstoff vorzugsweise in der Größenordnung von 0,2 bis 2 mm und meist nicht wesentlich über 1,2 mm. Die Klinge ist eben ausgeführt und fluchtet in jeder Seitenansicht mit dem hinteren Meißelteil 3. Die Meißelklinge ist breiter als der Meißelschaft.

Die Meißelführung 7 umfaßt einen flachen Führungsteil 8, der einen ebenen Führungsschlitz 9 enthält zur passenden Aufnahme der Meißelklinge 2. Während der flache Führungsteil 8 in seinem vorderen Abschnitt 10 rings geschlossen ausgeführt ist, weist er in seinem hinteren Teil 11 eine mittige Ausnehmung 12 zur Aufnahme des hinteren Meißelschafts 3 auf. Dieser wird durch die Kanten 13 der Ausnehmung 12 und die seitlich über ihn hinausstehenden, im Führungsschlitz 9 geführten Ränder 14 der Meißelklinge geführt. Gewünschtenfalls kann der flache Führungsteil 8 außerdem mit einem Rohrstück 15 verbunden sein, das hauptsächlich als Schutzrohr und zum Halten der Führung dient, das aber gewünschtenfalls auch mit seiner Innenfläche eine Führungsfläche für den hinteren Meißelteil bilden kann.

Die Schneide der Klinge ist, wie man in Fig. 3 sieht, auf beiden Flachseiten gleich und zur Mitte hin symmetrisch ausgeführt, damit sich ein mit der Klingenebene übereinstimmender Schnitt ergibt.

In Fig. 2 erkennt man die Scheide in zwei Relativstellungen gegenüber dem Meißel, nämlich in einer mit durchgezogenen Linien dargestellten Stellung, in welcher der Meißel in die Scheide zurückgezogen ist, während er in der durch strichpunktierte Linien angedeuteten Stellung gänzlich vorgeschoben ist.

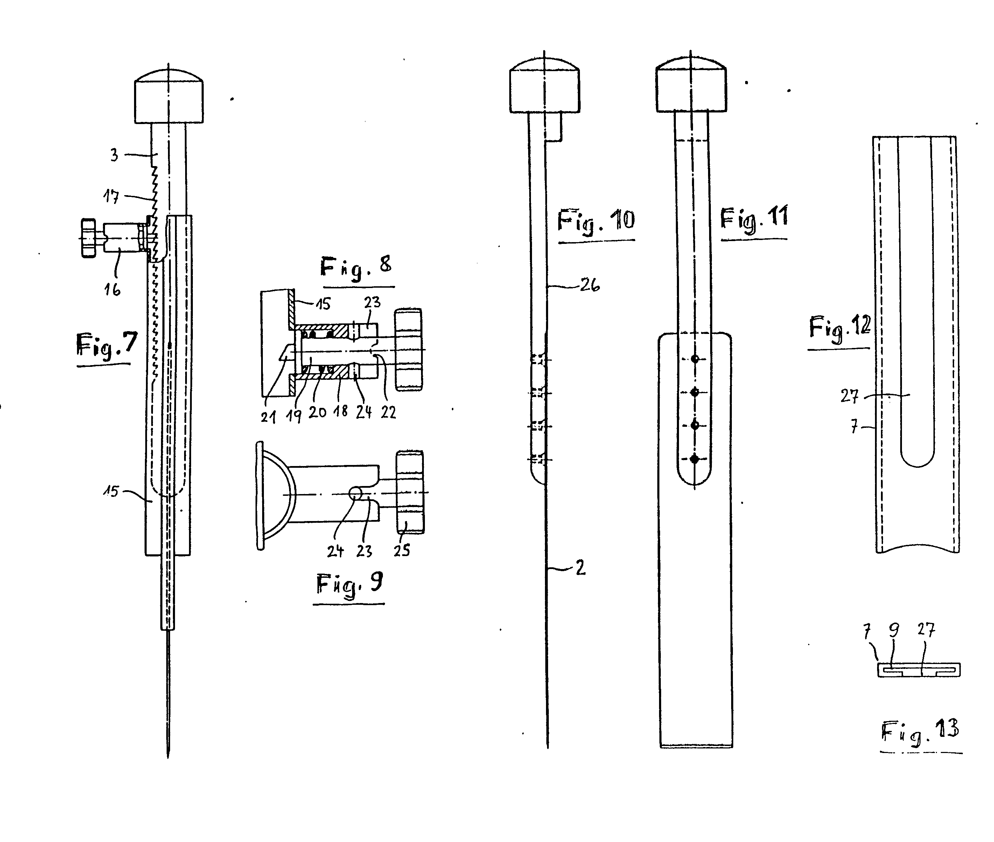

Das Ausführungsbeispiel gemäß Fig. 7 bis 9 stimmt mit demjenigen gemäß Fig. 1 bis 6 überein, soweit nicht im folgenden anderes angegeben ist. An dem Rohrstück 15 der Scheide ist seitlich eine Rasteinrichtung 16 angeordnet, die mit Rastzähnen 17 des hinteren Meißelteils 3 zusammenwirkt.

Wie man in Fig. 8 und 9 erkennt, besteht die Rasteinrichtung aus einer mit dem Rohrstück 15 fest verbundenen Fassung 18, in der ein Bolzen 19 quer zum Meißel verschiebbar gelagert ist und durch Federkraft 20 zum Meißel hingedrängt wird. Sein Ende 21 ist den sägezahnförmigen Rastzähnen des Meißels entsprechend gestaltet, wobei die Richtung der Sägezahnform so gewählt ist, daß beim Schlagen des Meißels die Scheide und beim Zurückziehen der Scheide der Meißel folgen muß. Die Fassung 18 bildet am äußeren Ende zwei senkrecht zueinander stehende Schlitze 22 und 23 unterschiedlicher Länge, die mit einem mit dem Bolzen 19 fest verbundenen Querbolzen 24 zusammenwirken. In der gezeigten Stellung liegt der Querbolzen 24 in der längeren Einbuchtung 23, wodurch die Bolzenspitze 21 mit der Rastzahnung des Meißels in Eingriff kommt. Zieht man den Bolzen 19 mittels des Griffes 25 zurück und dreht ihn um 90°, so daß der Querbolzen in Eingriff mit der Ausnehmung 22 kommt, so ist die Bolzenspitze 21 von den Rastzähnen des Meißels frei.

Die Ausführungsform des Meißels gemäß Fig. 10 und 11 entspricht derjenigen gemäß Fig. 3 und 4 mit Ausnahme des Umstands, daß der hintere Meißelteil auf einer Seite 26 fluchtend mit der zugehörigen Breitfläche der Klinge 2 abgeschnitten ist. Daher braucht die Scheide 7 nur auf einer Seite einen Ausschnitt 27 (entsprechend dem beidseitigen Ausschnitt 12 der oben beschriebenen Ausführungsform) aufzuweisen. Ein Schutzrohr 15 erübrigt sich in diesem Fall im allgemeinen, obwohl selbstverständlich auch diese Ausführung damit versehen sein könnte.

## Patentansprüche

1. Chirurgischer Meißel, dadurch gekennzeichnet, daß am vorderen Ende eines biegestarren Meißelschafts (3) eine langgestreckte, dünne, an ihrem vorderen Ende die Meißelschneide tragende Meißelklinge (2) richtungsgleich befestigt ist und daß der Meißelschaft (3) und die Meißelkklinge in Gleitführungen (9, 13, 15) einer Meißelführung (7) in ihrer Längsrichtung verschiebbar geführt sind.

2. Meißel nach Anspruch 1, dadurch gekennzeichnet, daß die Gleitführung (9) für die Meißelklinge (2) als Scheide (10) ausgebiltet ist.

3. Meißel nach Anspruch 1 oder 2, dadurch gekennzeichnet, daß gesonderte Gleitführungen (9, 13, 15) in der Meißelführung (7) für den Meißelschaft (3) und die Meißelklinge (2) vorgesehen sind.

4. Meißel nach einem der Ansprüche 1 bis 3, dadurch gekennzeichnet, daß der Meißelschaft (3) in der Meißelführung (7) arretierbar ist.

## Claims

1. A surgical tool, characterised in that a thin elongate tool blade (2) carrying the tool cutting edge at its front end is secured equidirectionally to the front end of a rigid tool shank (3), and in that the tool shank (3) and the tool blade are guided displaceably in their longitudinal direction in sliding guides (9, 13, 15) of a tool guide (7).

2. A tool according to Claim 1, characterised in that the sliding guide (9) for the tool blade is in the form of a sheath (10).

3. A tool according to Claim 1 or 2, characterised in that separate sliding guides (9, 13, 15) are provided in the tool guide (7) for the tool shank (3) and the tool blade (2).

4. A tool according to any of Claims 1 to 3, characterised in that the tool shank (3) can be located in the tool guide (7).

## Revendications

1. Ciseau chirurgical, caractérisé en ce qu'il est fixé dans la même direction, à l'extrémité avant d'une tige de ciseau (3) rigide à la flexion, une lame de ciseau (2) mince, de forme allongée, portant le tranchant de ciseau à son extrémité avant, et en ce que la tige de ciseau (3) et la lame de ciseau sont guidées dans des coulisses (9, 13, 15) d'un guide-ciseau (7), de manière à pouvoir se déplacer dans leur directien longitudinale.

2. Ciseau selon la revendication 1, caractérisé en ce que la coulisse (9) peur la lame de ciseau (2) est réalisée sous forme de fourreau (10).

3. Ciseau selen la revendication 1 ou 2, caractérisé en ce que des coulisses séparées (9, 13, 15) sont prévues dans le guide-ciseau (7) pour la tige de ciseau (3) et pour la lame de ciseau (2).

4. Ciseau selon l'une quelconque des revendications 1 à 3, caractérisé en ce que la tige de ciseau (3) peut être arrêtée dans le guide-ciseau (7).

Fig.1

Fig.2

Fig.3

Fig.4

Fig.5

Fig.6

4

3

1

15

2

10

8
7

2

2

3

5

1

6

2

4

14

8
13

7
11
15

10

8 9 15 13

12

0157172

Fig.12

Fig.13

Fig.11

Fig.10

Fig.8

Fig.9

Fig.7